# EUROPÄISCHE PATENTSCHRIFT

(11) **EP 1 192 972 B1**
(45) Veröffentlichungstag und Bekanntmachung des Hinweises auf die Patenterteilung: **14.06.2006**
(21) Anmeldenummer: 01250337.1
(22) Anmeldetag: 25.09.2001
(51) Int. Cl.: A61N 1/372

(54) **Elektrisch aktives Implantat**
Electrical active implant
Implant électriquement actif

(30) Priorität: 28.09.2000 DE 10049727
(43) Veröffentlichungstag der Anmeldung: 03.04.2002
(73) Patentinhaber: BIOTRONIK GmbH & Co. KG, 12359 Berlin (DE)
(72) Erfinder: Blömer, Frank, 12163 Berlin (DE); Schaldach, Max, 91054 Erlangen (DE)
(74) Vertreter: Lindner-Vogt, Karin L.

(56) Entgegenhaltungen:
- EP-A- 0 545 242
- EP-A- 0 560 470
- WO-A-00/59376
- WO-A-01/35813
- WO-A-95/16313
- FR-A- 2 781 905
- US-A- 5 080 096
- US-A- 5 350 407
- US-A- 5 522 856

## Beschreibung

Die vorliegende Erfindung betrifft einer implantierbaren Herzschrittmacher oder einen implantierbaren Defibrillator welcher energieverbrauchende Komponenten und Aktiviermittel zum Aktivieren des Implantats aufweist in Kombination mit einer Verpackung, die zuzätzlich einen Magneten enthält.

Nach der Fertigstellung eines Implantats wie beispielsweise eines Herzschrittmacher oder eines Defibrillators und vor seiner Implantation wird das Implantat üblicherweise gelagert. Dabei beträgt bespielsweise der typische Stromverbrauch eines sich im Lager befindlichen Schrittmachers ca. 8 µA nach der Implantation hingegen abhängig von Programmierung und Grad der Inhibierung zwischen 12 und 22 µA. Daraus folgt, dass zwei Jahre Lagerzeit ungefähr einem Jahr Implantationszeit entsprechen. Der während einer Lagerung verbrauchte Strom kann folglich nicht mehr während der Implantationszeit genützt werden, so dass die tatsächliche Nutzungsdauer des Implantats, nämlich die Implantationszeit, herabgesetzt wird.

Das derzeitige Use-Before-Datum, d.h. dasjenige Datum, vor welchem das Implantat in Betrieb genommen werden muss, richtet sich nach dem Datum des Batterieanschlusses, dem Sterilisationsdatum, einer produktspezifischen Zeitspanne und berücksichtigt ferner den Stromverbrauch während einer Lagerung. Nach Ablauf des Use-Before-Datums werden nicht implantierte Geräte zur Resterilisation an den Hersteller zurückgeschickt. Dabei kann es vorkommen, dass aufgrund der während einer Lagerung entnommenen Ladung Implantate nicht mehr regulär als Verkaufsprodukte ausgeliefert werden können. Hierbei sind abgesehen vom Batteriezustand im wesentlichen keine Gründe vorhanden, die einen maximalen Use-Before-Zeitraum von beispielsweise 24 Monaten erzwingen. Der Use-Before-Zeitraum könnte durch Reduzierung des während einer Lagerung verbrauchten Stromes ausgedehnt werden, wodurch die Anzahl der zur Resterilisation zurückgelieferten Implantate sowie die Anzahl der zu verschrottenden Einheiten reduziert werden könnte.

Die Funktion von Herzschrittmachern und insbesondere die aller Defibrillatoren ist temperaturabhängig. Wenn während der Lagerung oder eines Transportes Temperaturen unterhalb von beispielsweise 5°C auftreten, so ist mit einem Auftreten meist reversibler aber auch irreversibler Fehler zu rechnen. Derart geschädigte Einheiten können in aller Regel nicht mehr regulär ausgeliefert werden, da der Stromverbrauch nach Auftreten des Fehlers und somit der Batteriezustand nicht hinreichend genau bestimmt werden kann.

Aus dem Stand der Technik sind elektrische Implantate bekannt, die während einer Lagerung teilweise in eine stromsparende Stand-By-Betriebsart versetzt werden. Dabei werden üblicherweise bestimmte Komponenten des Implantats abgeschaltet, während andere Komponenten weiterhin mit Strom versorgt werden. Diese Stand-By-Betriebsart soll der Reduzierung des während einer Lagerung verbrauchten Stromes dienen.

Aus US-5,522,856 ist ein Implantat bekannt, welches ausgewählte Schaltungselemente des Implantats in eine stromsparende Betriebsart versetzt, bis das Implantat in einen Patienten implantiert wird. Das bekannte Implantat weist ferner einen Detektor auf, der bestimmt, ob das Implantat zur Implantierung angeschlossen wird. Wenn eine derartige Implantierung detektiert wird, werden die Schaltungselemente des Schrittmachers in die normale stromverbrauchende Betriebsart versetzt. Das Implantat kann ferner aus dem Lager entnommen werden und hinsichtlich seiner Funktion getestet werden und dann wieder in das Lager zurückgebracht werden, wobei das Implantat dabei wieder in eine stromsparende Stand-By-Betriebsart versetzt wird, um den Stromverbrauch während der Lagerung zu reduzieren. Des weiteren erfasst das Implantat, wenn die Impedanz an den Elektroden unter einen vorbestimmten Pegel fällt. Wenn dies aufgetreten ist, verbleibt das Implantat permanent in der normalen Betriebsart.

US 5,350,407 offenbart ein Implantat, welches sowohl in einen Wartezustand in Abhängigkeit von einer Anweisung von einer externen Kommunikationsvorrichtung als auch in einen aktiven Zustand in Abhängigkeit von einem Entfernen eines Aktivierungsstiftes oder in Abhängigkeit von einer Aktivierungsanweisung von einer externen Kommunikationsvorrichtung betreibbar ist. Nach der Fertigung des Implantats wird der Aktivierungsstift in das Implantat eingefügt und die externe Kommunikationsvorrichtung wird getriggert, um eine Deaktivierungsanweisung an das Implantat zu übermitteln. Dabei wird die Stromversorgung für einen Teil der Schaltungselemente des Implantats abgeschaltet, während die Stromversorgung für diejenigen Schaltungselemente aufrechterhalten werden, die zur Aktivierung der restlichen Schaltungselemente in Verbindung mit dem Kommunikationsbetrieb erforderlich sind. Das Implantat wird dabei entweder durch eine Übertragung einer Aktivieranweisung von einer externen Kommunikationsvorrichtung oder durch Entfernen des Aktivierungsstiftes aktiviert.

Die beiden genannten Druckschriften offenbaren folglich bereits eine Reduzierung des Stromverbrauches des Implantates während seiner Lagerung. Es ist jedoch wünschenswert, den Stromverbrauch des Implantates während einer Lagerung weiter zu reduzieren.

Das Dokument WO-A-00/59376 gehört zum Stand der Technik gemäß Artikel 54(3) EPC. Der Gegenstand des Anspruchs 1 unterscheider sich von der Offenbarung dieses Dokuments dadurch, dass das Implantat einen Herzschrittmacher oder Defibrillator darstellt.

Es ist daher Aufgabe der Erfindung, ein elektrisch aktives Implantat derart weiterzubilden, dass der Stromverbrauch des Implantates während seiner Lagerung im Vergleich zu dem Stand der Technik weiter reduzieren kann.

Diese Aufgabe wird durch eine Vorrichtung gemäß dem Anspruch 1 gelöst.

Der Erfindung liegt dabei der Gedanke zugrunde, ein elektrisch aktives Implantat mit energie-verbrauchenden Komponenten und mit Aktiviermittel zum Aktivieren des Implantates vorzusehen. Ferner weist das elektrisch aktive Implantat Deaktiviermittel zum Deaktivieren der energie-verbrauchenden Komponenten des Implantates auf, wobei die energie-verbrauchenden Komponenten des Implantats während seiner Lagerung mittels des Deaktiviermittels deaktivierbar sind. Ferner weist das Implantat ein Mittel zum nicht-flüchtigen Speichern von implantat-spezifischen Daten auf.

Die mit der Erfindung einhergehenden Vorteile bestehen insbesondere darin, dass während der Lagerhaltung alle energie-verbrauchenden Komponenten des Implantates deaktiviert bzw. abgeschaltet werden können, so dass kein Strom im dem Implantat während seiner Lagerung fließt. Ferner wird sichergestellt, dass notwendige implantat-spezifische Daten auch bei einer Abschaltung aller energieverbrauchenden Komponenten des Implantates erhalten bleiben.

Eine Ausgestaltung der Erfindung sieht vor, dass die Mittel zum Speichern einen nicht-flüchtigen elektronischen Speicher darstellen, in dem die implantat-spezifischen Daten nach der Herstellung des Implantates in den elektronischen nicht-flüchtigen Speicher eingeschrieben werden. Die in diesem elektronischen Speicher gespeicherten Daten lassen sich ohne Probleme nach Aktivierung des Implantates wieder auslesen.

Bei einer weiteren Ausgestaltung der Erfindung enthält das Mittel zum Speichern einen auf dem Implantat abgebildeten Strichcode, welcher die implantat-spezifischen Daten beinhaltet. Auch hier lassen sich die implantat-spezifischen Daten wieder nach der Aktivierung des Implantates extrahieren. Alternativ dazu kann der implantat-spezifische Daten enthaltende Strichcode auch an der Verpackung des Implantates angebracht werden.

Das Aktiviermittel weist mindestens einen drahtlos aktivierbaren Schalter auf, welcher zur Reaktivierung der deaktivierten energie-verbrauchenden Komponenten dient. Dieser Schalter wird dabei magnetisch aktiviert werden. Mit Hilfe des drahtlos aktivierbaren Schalters kann die Reaktivierung ohne ein Eingreifen in das Implantat durchgeführt werden.

Bei einer weiteren Ausgestaltung der Erfindung wird der sich auf dem Implantat oder auf der Verpackung des Implantats befindlichen Strichcode von einem externen Barcode-Scanner ausgelesen, wonach die implantat-spezifischen Daten extrahiert werden und mittels eines externen telemetrischen Senders an das Implantat übermittelt werden. Die übermittelten implantat-spezifischen Daten werden in einem Speicher in dem Implantat gespeichert. Auf diese Weise können die implantat-spezifischen Daten im Nachhinein in dem Implantat gespeichert werden.

Die erfindungsgemäße Ausführungsform umfasst ein Implantat mit einem magnetisch aktivierbaren Schalter und einer Verpackung, die einen Magneten umfasst, welcher so angeordnet ist, dass der magnetisch aktivierbare Schalter des Implantates immer dann aktiviert wird, wenn das Implantat der Verpackung entnommen wird. Dies ermöglicht ein automatisches Aktivieren des Implantates bei der Entnahme aus der Verpackung vor dem Implantieren.

Bei einer weiteren Ausgestaltung der Erfindung weist das Implantat ein Reaktivier-Sperrmittel zum Sperren von wiederholten Aktivierungen bzw. Deaktivierung der energie-verbrauchenden Komponenten des Implantates auf. Dadurch soll verhindert werden, dass das Implantat wiederholt unautorisiert aktiviert bzw. deaktiviert wird.

Weitere Ausgestaltungen der Erfindung sind Gegenstand der Unteransprüche.

Nachfolgend werden Ausführungsbeispiele der Erfindung anhand der beigefügten Zeichnung beschrieben.

Es zeigen dabei:
- Figur 1: ein schematisches Blockschaltbild eines Implantates gemäß einem ersten Ausführungsbeispiel,
- Figur 2: ein schematisches Blockschaltbild eines Implantates gemäß einem zweiten Ausführungsbeispiel, und
- Figur 3: eine Darstellung des Zusammenwirkens der Komponenten des zweiten Ausführungsbeispiels.

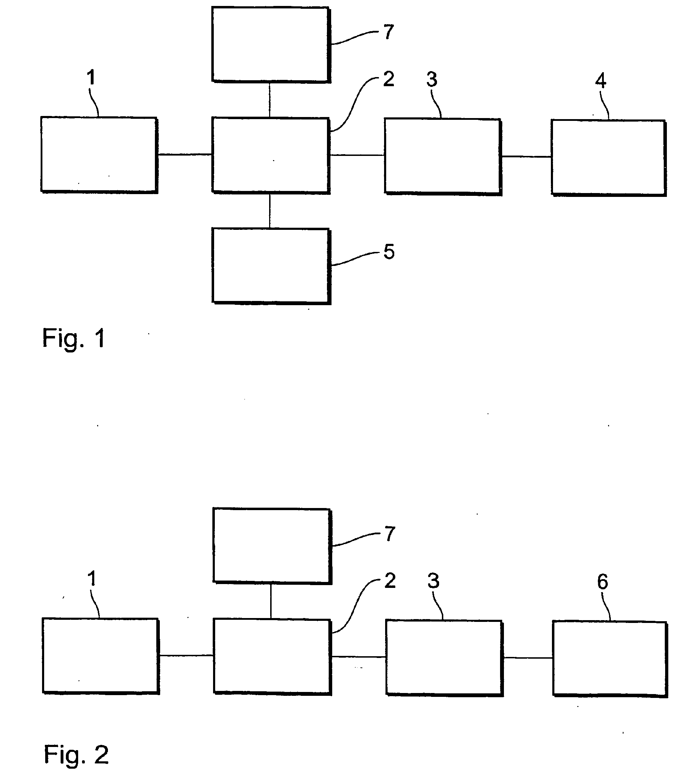

In Figur 1 ist ein erstes Ausführungsbeispiel eines elektrisch aktiven Implantates gezeigt. Das Implantat weist eine Batterie 1, Aktivier-/Deaktiviermittel 2, ein Reaktivier-Sperrmittel 5, welches mit dem Aktivier-/Deaktiviermittel 2 verbunden ist, herkömmliche energie-verbrauchende Komponenten 3 des Implantates, sowie einen nicht-flüchtigen elektronischen Speicher 4 auf.

Nach der Fertigung des Implantates werden implantat-spezifische Daten wie beispielsweise die Seriennummer des Implantates und das Werksausgangsprogramm in dem nicht-flüchtigen elektronischen Speicher 4 gespeichert. Die in dem Speicher 4 gespeicherten Daten bleiben auch bei einer Abschaltung der Strom- und Spannungsversorgung erhalten, so dass sie nach einer Aktivierung des Implantates wieder verwendet werden können

Nach der Werksausgangsprüfung des Implantates werden alle stromverbrauchenden Komponenten wie beispielsweise die CPU, Ladungspumpen, Ausgangsstufen oder dergleichen deaktiviert bzw. ausgeschaltet. Diese Implantate werden dann in diesem stromlosen Zustand gelagert. Damit wird erreicht, dass der Stromverbrauch des Implantates während einer Lagerung sehr stark reduziert wird und gegen Null geht.

Durch die stromlose Lagerung des Implantates kann die Implantationszeit, d.h. die Zeit, während der das Implantat in dem Körper implantiert wird, verlängert werden.

Ferner kann der temperaturbedingte Einfluß während der Lagerung oder des Transports durch die Deaktivierung der stromverbrauchenden Komponenten nahezu ausgeschlossen werden.

Wenn das gelagerte Implantat in einen Körper implantiert werden soll bzw. wenn das Implantat bereits implantiert ist, erfolgt die Aktivierung der energieverbrauchenden Komponenten 3. Die Aktivierung wird mittels eines kleinen sich in der Blisterpackung befindlichen Magneten durchgeführt, welcher einen im Implantat befindlichen REED-Umschalter betätigt. Bei der Entnahme des Implantates aus der Blisterpackung schließt der REED-Schalter und aktiviert beispielsweise die Schrittmacherfunktion. In dieser Funktion bzw. Betriebsart arbeitet das Implantat im Normalbetrieb. Mit anderen Worten, die energie-verbrauchenden Komponenten 3 des Implantates werden durch die Batterie 1 mit Strom versorgt, so dass das Implantat alle Funktionen wahrnehmen kann und auf die in dem Speicher 4 gespeicherten implantat-spezifischen Informationen zurückgreifen kann.

Um nach der erfolgten Aktivierung eine nochmalige Deaktivierung durch Öffnen des REED-Schalters bei einer späteren Auflage eines Magneten zu verhindern, weist das Implantat ein Reaktivier-Sperrmittel 5 auf. Dieses Reaktivier-Sperrmittel 5 steuert die Schaltungen des Implantates derart, dass eine nochmalige Deaktivierung durch den Anwender ausgeschlossen ist und nur durch den Ansteller unter Verwendung eines speziellen telemetrischen Senders möglich ist.

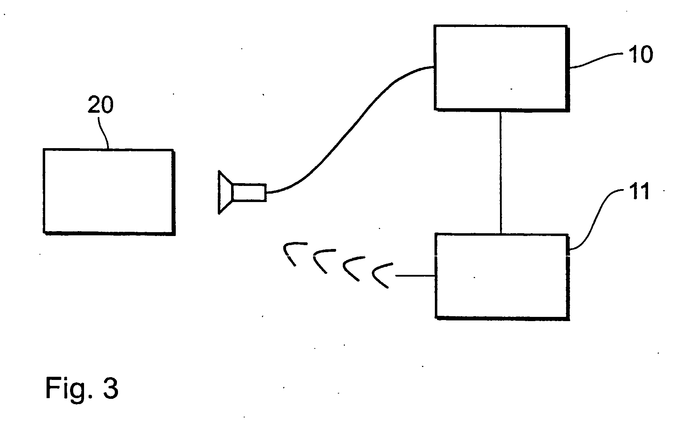

In den Figuren 2 und 3 ist ein Implantat gemäß einem zweiten Ausführungsbeispiel (Fig. 2) und das Zusammenwirken seiner Komponenten (Fig. 3) gezeigt. Das Implantat weist eine Batterie 1, ein Aktivier-/Deaktiviermittel 2, energieverbrauchende Komponenten 3, ein Reaktivier-Sperrmittel 5 sowie einen Speicher 6 auf. Bei diesem Ausführungsbeispiel werden keine Daten nach der Werksausgangsprüfung im Implantat gespeichert. Das Implantat wird vollständig abgeschaltet, so dass es während seiner Lagerung keinen Strom verbraucht.

Die implantat-spezifischen Daten werden kodiert und in einem Strichcode umgewandelt. Dieser Strichcode wird dann an dem Implantat oder an der Verpackung des Implantats angebracht.

Nach der Fertigung und der Verpackung des Implantates ist es durch einen entsprechenden telemetrischen Befehl möglich, die Batterie 1 von den energieverbrauchenden Komponenten 3 des Implantates mit Hilfe des Aktivier-/Deaktiviermittels 2 zu trennen.

Bevor das Implantat an den Endkunden ausgeliefert wird, wird das Implantat durch eine Programmiergerät initialisiert. Dieses externe Programmiergerät 12 weist gemäß Figur 3 einen Barcode-Scanner 10 und einen telemetrischen Sender 11 auf. Der Barcode-Scanner liest den sich auf dem Implantat oder auf der Verpackung des Implantats befindlichen Strichcode ein, extrahiert die implantat-spezifischen Daten und sendet diese Daten telemetrisch an das Implantat, wo diese in dem Speicher 6 zur weiteren Verwendung abgelegt werden. Die von dem Sender 11 übertragenen Informationen werden von einem Empfänger 7 des Implantates empfangen und an den Speicher 6 weitergeleitet, wo sie gespeichert werden.

Das Einlesen des Strichcodes und das Übermitteln der extrahierten implantat-spezifischen Daten soll wie folgt geschehen:
1. Lesen der Barcode-Daten
2. Extraktion der implantarspezifischen Daten
3. Übermittlung der Daten an das Implantat
4. Rücksendung der im Implantat abgelegten Daten
5. Vergleich der gesendeten mit den rückgesendeten Daten
6. Bestätigung der korrekten Initialisierung, wenn gesendete und rückgesendete Daten identisch.

Während der Schritte 1 bis 6 wird permanent der Barcode gescannt. Tritt eine Veränderung auf (z.B. Entfernen des Barcodes), so wird der Initialisierungsvorgang sofort und ohne Bestätigung abgebrochen.

Dies soll verhindern, dass die gescannten Daten eines Implantates in einem anderen Implantat abgelegt werden.

Die Aktivierung des Implantates nach der Auslieferung erfolgt ähnlich wie bei dem Ausführungsbeispiel der Fig. 1 durch eine Magnetauflage, die einen REED-Schalter schließt, wodurch die energieverbrauchenden Komponenten 3 des Implantates an die Batterie 1 angekoppelt werden, um so mit Strom und Spannung versorgt zu werden.

Nach der vollständigen Initialisierung wird eine dauerhafte Überbrückung des REED-Schalters beispielsweise durch ein Thermo-Element erreicht. Das Thermo-Element kann dabei als Bi-Metall-Schalter ausgestaltet sein. Durch die dauerhafte Überbrückung kann eine fälschliche Deaktivierung im implantierten Zustand ausgeschlossen werden.

Eine derartige Überbrückung des REED-Schalters kann selbstverständlich auch in dem ersten Ausführungsbeispiel durchgeführt werden.

Alternativ zu dem ersten und zweiten Ausführungsbeispiel kann das Werksausgangsprogramm sowie die Seriennummer des Implantates in einem separaten Speicher des Implantates abgelegt werden, welcher kontinuierlich mit Spannung versorgt wird. Alle anderen energie-verbrauchenden Komponenten des Implantates wie beispielsweise CPU, Ladungspumpen, Ausgangsstufen oder dergleichen, werden nach der Werksausgangsprüfung komplett deaktiviert bzw. abgeschaltet.

Während der Lagerung des Implantates verbraucht dabei lediglich der Speicher für das Werksausgangsprogramm und die Seriennummer Strom, während alle anderen Komponenten des Implantates abgeschaltet sind.

Für die Aktivierung des Implantates vor der Implantierung sind mehrere Möglichkeiten vorhanden. Wie in dem ersten Ausführungsbeispiel beschrieben, wird ein sich in dem Implantat befindlicher REED-Schalter durch einen kleinen Magneten betätigt, welcher sich in dem Blister befindet. Bei der Entnahme des Implantates aus dem Blister schließt der REED-Schalter und aktiviert somit die normale Betriebsart des Implantates, indem die energieverbrauchenden Komponenten des Implantates an die Batterie gekoppelt werden, wodurch sie mit Strom und Spannung versorgt werden. Eine nochmalige Deaktivierung des Implantates durch Öffnen des REED-Schalters durch einen weitere Auflage eines Magneten wird durch ein Reaktivier-Sperrmittel verhindert.

Die Aktivierung kann ferner durch einen Stift im Implantat erfolgen. Dieser Stift bzw. Stecker bewirkt bei seiner Entnahme vor der Implantation, dass in dem Implantat der ventrikuläre differente Kontakt mit dem indifferenten Kontakt kurzzeitig niederohmig verbunden wird. Dieses Signal wird von der Steuerelektronik des Implantates zur dauerhaften Aktivierung der vollen Funktionalität verwendet.

Ferner kann die Aktivierung des Implantates erfolgen, indem eine liegende Elektrode an das Implantat angeschlossen ist. Die Elektronik des Implantates erfasst eine externe Last, welche kleiner als 3200 Ohm ist, wodurch die volle Funktionalität dauerhaft aktiviert wird.

## Patentansprüche

1. Verpackung mit Implantat, wobei das Implantat energie-verbrauchende Komponenten (3), Aktiviermittel (2) zum Aktivieren des Implantats, und Deaktiviermittel (2) zum Deaktivieren der energie-verbrauchenden Komponenten (3) des Implantats aufweist wobei das Implantat ein Herzschrittmacher oder ein Defibrillator ist und die energie-verbrauchenden Komponenten (3) des Implantats während seiner Lagerung mittels des Deaktiviermittels (2) deaktivierbar sind, und wobei das Implantat ein Mittel (4) zum nicht-flüchtigen Speichern von implantat-spezifischen Daten aufweist wobei das Aktiviermittel (2) mindestens einen magnetisch aktivierbaren Schalter aufweist, welcher zur Reaktivierung der deaktivierten energie-verbrauchenden Komponenten (3) dient und die Verpackung einen Magneten enthält, der so angeordnet ist, dass der Schalter beim Entnehmen des Herzschrittmachers oder Defibrillators aus der Verpackung aktiviert wird.

2. Vorrichtung nach Anspruch 1, **dadurch gekennzeichnet, dass** das Mittel zum Speichern (4) als nicht-flüchtiger elektronischer Speicher (4) ausgestaltet ist, wobei die implantat-spezifischen Daten nach der Herstellung das Implantats in dem elektronischen nicht-flüchtigen Speicher (4) einschreibbar sind.

3. Vorrichtung nach Anspruch 1, **dadurch gekennzeichnet, dass** das Mittel zum Speichern einen auf dem Implantat abgebildeten Strichcode enthält, welcher implantat-spezifische Daten beinhaltet.

4. Vorrichtung nach Anspruch 1 , **dadurch gekennzeichnet, dass** das Speichermittel einen implantat-spezifische Daten enthaltenden Strichcode enthält, welcher an der Verpackung des Implantat anbringbar ausgestaltet ist.

5. Vorrichtung nach Anspruch 3 oder 4 und Barcode-Scanner (10) **dadurch gekennzeichnet, dass** der Barcode-Scanner (10) zum Lesen des Strichcodes und zum Extrahieren der Implantat-spezifischen Daten ausgebildet sowie mit einem externen telemetrischen Sender (11) verbunden ist, um die Implantat-spezifischen Daten an das Implantat zu übermitteln, wobei das Implantat einen zweiten Speicher (6) zum Speichern der übermittelten Implantat-spezifischen Daten aufweist.

6. Vorrichtung nach einem der Ansprüche 1 bis 5, **gekennzeichnet durch** ein Reaktivier-Sperrmittel (5) zum Sperren von wiederholten Reaktivierungen der energie-verbrauchenden Komponenten (3) des Implantats.

7. Vorrichtung nach einem der vorherigen Ansprüche, **dadurch gekennzeichnet, dass** das Deaktiviermittel (2) mindestens einen drahtlos aktivierbaren Schalter aufweist.

8. Vorrichtung nach einem der vorherigen Ansprüche, wobei das Aktiviermittel und das Deaktiviermittel als ein einzelnes Mittel (2) ausgebildet ist.

## Claims

1. A package having an implant, the implant having energy-consuming components (3), activation means (2) for activating the implant, deactivation means (2) for deactivating the energy-consuming components (3) of the implant, the implant being a pacemaker or a defibrillator and the energy-consuming components (3) of the implant being deactivatable during its storage using the deactivation means (2), and the implant having means (4) for non-volatile storage of implant-specific data, the activation means (2) having at least one magnetically activatable switch, which is used for reactivating the deactivated energy-consuming components (3) and the package containing a magnet, which is positioned in such way that the switch is activated when the pacemaker or defibrillator is removed from the package.

2. The device according to Claim 1, **characterized in that** the means for storage (4) is implemented as a non-volatile electronic memory (4), the implant-specific data being writable in the electronic non-volatile memory (4) after the manufacturing of the implant.

3. The device according to Claim 1, **characterized in that** the means for storage contain a barcode imaged on the implant, which contains implant-specific data.

4. The device according to Claim 1, **characterized in that** the storage means contains a barcode containing implant-specific data, which is implemented as attachable to the package of the implant.

5. The device according to Claim 3 or 4 and a barcode scanner (10), **characterized in that** the barcode scanner (10) is implemented for reading the barcode and for extracting the implant-specific data and is connected to an external telemetric transmitter (11), in order to transmit the implant-specific data to the implant, the implant having a second memory (6) for storing the transmitted implant-specific data.

6. The device according to one of Claims 1 through 5, **characterized by** a reactivation blocking means (5) for blocking repeated reactivations of the energy-consuming components (3) of the implant.

7. The device according to one of the preceding claims, **characterized in that** the deactivation means (2) has at least one wirelessly activatable switch.

8. The device according to one of the preceding claims, wherein the activation means and the deactivation means are implemented as a single means (2).

## Revendications

1. Emballage avec implant, dans lequel l'implant comporte des composants consommateurs d'énergie (3), un moyen d'activation (2) pour activer l'implant et un moyen de désactivation (2) pour désactiver les composants consommateurs d'énergie (3) de l'implant, l'implant étant un pacemaker cardiaque ou un défibrillateur et les composants consommateurs d'énergie (3) de l'implant étant désactivables au choix pendant son stockage au moyen du moyen de désactivation (2), dans lequel l'implant comporte un moyen (4) de sauvegarde non volatile de données spécifiques à l'implant, le moyen d'activation (2) présente au moins un interrupteur activable magnétiquement qui sert à réactiver les composants consommateurs d'énergie (3) désactivés et l'emballage contient un aimant qui est disposé de manière à ce que l'interrupteur soit activé lors du retrait du pacemaker cardiaque ou du défibrillateur de l'emballage.

2. Dispositif selon la revendication 1, **caractérisé en ce que** le moyen de sauvegarde (4) est réalisé sous forme d'une mémoire électronique non volatile (4), dans lequel les données spécifiques à l'implant sont inscriptibles après fabrication de l'implant dans la mémoire électronique non volatile (4).

3. Dispositif selon la revendication 1, **caractérisé en ce que** le moyen de sauvegarde contient un code barres apposé sur l'implant et contenant des données spécifiques à l'implant.

4. Dispositif selon la revendication 1, **caractérisé en ce que** le moyen de sauvegarde contient un code barres contenant des données spécifiques à l'implant et qui est conçu de manière à pouvoir être apposé sur l'emballage de l'implant.

5. Dispositif selon la revendication 3 ou 4 et scanner de code barres (10), **caractérisés en ce que** le scanner de code barres (10) est conçu pour lire le code barres et pour extraire les données spécifiques à l'implant et est relié à un émetteur télémétrique externe (11) afin de transmettre les données spécifiques à l'implant à l'implant, tandis que l'implant présente une deuxième mémoire (6) pour la sauvegarde des données spécifiques à l'implant transmises.

6. Dispositif selon une des revendications 1 à 5, **caractérisé par** un moyen d'arrêt de réactivation (5) pour arrêter les réactivations répétées des composants consommateurs d'énergie (3) de l'implant.

7. Dispositif selon une des revendications précédentes, **caractérisé en ce que** le moyen de désactivation (2) comporte au moins un interrupteur activable sans fil.

8. Dispositif selon une des revendications précédentes, **caractérisé en ce que** le moyen d'activation et de désactivation est conçu sous forme d'un moyen unique.
